# EUROPEAN PATENT SPECIFICATION

(11) **EP 0 632 692 B1**
(45) Date of publication and mention of the grant of the patent: **12.06.1996**
(21) Application number: 93907932.3
(22) Date of filing: 26.03.1993
(51) Int. Cl.: A01N 25/08, A01N 25/14, A01N 25/34, A61K 9/20, A61K 9/16

(54) **DISPERSIBLE PRODUCTS**
DISPERGIERBARE PRODUKTE
PRODUITS DISPERSIBLES

(30) Priority: 28.03.1992 GB 9206821
(43) Date of publication of application: 11.01.1995
(73) Proprietor: BORDEN (UK) LIMITED, Southampton S052 9ZB (GB)
(72) Inventor: DODD, Stephen Francis, Nr St Asaph, Clwyd LL17 0HG (GB)
(74) Representative: Wilkinson, Stephen John
(86) International application number: GB9300629
(87) International publication number: WO9319596

(56) References cited:
- WO-A-90/08470
- CH-A- 639 635
- GB-A- 1 512 637
- GB-A- 2 081 703
- CHEMICAL PATENTS INDEX, DOCUMENTATION ABSTRACTS JOURNAL Section Ch, Week 9121, Derwent Publications Ltd., London, GB; Class C, AN 153080
- CHEMICAL PATENTS INDEX, BASIC ABSTRACTS JOURNAL Section Ch, Week 8743, Derwent Publications Ltd., London, GB; Class C, AN 304295
- CHEMICAL PATENTS INDEX, DOCUMENTATION ABSTRACTS JOURNAL Section Ch, Week 9006, Derwent Publications Ltd., London, GB; Class C, AN 040547
- PESTICIDE SCIENCE, vol. 9, no. 5, October 1978, Barking (GB); C.F. DRAKE, p. 441

## Description

This invention relates to products which are dispersed by an aqueous medium. More particularly it relates to products which have a component that dissolves in an aqueous medium, the remaining product being dispersed by the aqueous medium. Furthermore it relates to a product in which the dissolving of the component and the dispersal of the remaining product is effected in a controlled manner in an aqueous medium.

In some industries an active material is released into an environment in order to effect a controlled reaction in the environment. The active material is commonly stored as a solid or as solution, and usually has to be part of an aqueous medium to be released.

In the agricultural industry crops of cultivated plants are protected by the use of pesticides and/or insecticides. Hereinafter the term "pesticide" includes insecticides and other such materials. Pesticides are usually stored in liquid form and sprayed directly onto the crop to destroy insects and/or other organisms which are harmful to the crop. The pesticide may be sold as a concentrated liquid which needs to be diluted prior to spraying of the crop.

Some pesticides seriously contaminate the material of the container in which they are held and this can lead to problems in the disposal of these containers. In some cases, the contamination is so bad that the containers are returned to the pesticide manufacturer who will arrange for proper disposal of the contaminated containers. This is significantly costly to the manufacturer and consequently there is a need to reduce or substantially eliminate the cost of contamination of the packaging of pesticides.

In the pharmaceutical industry many drugs are administered by mixing a solid (i.e. a tablet) which contains an active material with an aqueous medium (i.e. water) which is then swallowed by the patient. Some tablets dissolve or break up to form a suspension in the water, and others are swallowed whole with the water being `used as an aid to swallowing the tablet.

It will be appreciated that it is necessary to carefully control the dissolution rate or break-up rate of tablets which contain drugs depending on which drug is to be administered. Some tablets take a considerable time to fully dissolve or break up which is inconvenient and extends the discomfort felt by the patient. Consequently there is a need to control the dissolution rate or break-up rate of some pharmaceutical tablets.

According to the present invention there is provided a product which disperses in a controlled manner in an aqueous medium, said product comprising at least an active material and a binder, the binder including one or more of polyphosphate chains, borate ions, or silicate ions.

Preferably the polyphosphate chains, or borate ions or silicate ions have been respectively derived from at least one water soluble phosphate and/or borate and/or silicate glass.

In one embodiment of the present invention the binder is mixed with the active material in the form of an aqueous solution of the at least one water soluble glass. In a further embodiment of the present invention particulate binder is mixed with the active material in the form of particles of the at least one water soluble glass and the polyphosphate chains and/or borate ions and/or silicate ions have been formed by mixing water with the mixture of active material and glass particles. The glass particles may be wholly or partially dissolved into the water thereby to form the polyphosphate chains and/or borate ions and/or silicate ions.

In a further embodiment of the invention the binder comprises crystalline phosphate.

Without wishing to be bound by theory it is believed that the polyphosphate chains are formed following the dissolution of the respective water soluble glasses into aqueous solution. These chains form an interlinking matrix throughout the product which is enhanced by hydrogen bonding of the chains by the chemically bonded water molecules therein. After removal of excess water the resulting dried product retains the polyphosphate matrix which firmly binds together the active material.

Preferably the binder comprises at least 0.25% by weight and the active material comprises up to 99.75% by weight of the total weight of the product. More preferably the binder comprises from 0.5 to 50% by weight and the active material comprises from 99.5 to 50% by weight of the total weight of the product.

As described hereinabove, it is believed that the polyphosphate chains and/or borate ions or silicate ions form an interlinking matrix which may additionally include hydrogen bonding by chemically bonded water molecules. Generally, full removal of chemically bound water is undesirable as this would destroy the hydrogen bonding and thus weaken the structure.

Preferably, the water soluble phosphate glass comprises from 30 to 80 mol% P₂O₅, from 20 to 70 mol% R₂O, from 0 to 30 mol% MO and from 0 to 15 mol% L₂O₃, where R is Na, K or Li, M is Ca, Mg or Zn and L is Al, Fe or B. More preferably, the water soluble phosphate glass comprises from 58 to 72 wt% P₂O₅, from 42 to 28 wt% Na₂O and from 0 to 16 wt% CaO.

Such glasses include glasses of the following compositions in weight %:

| | 1 | 2 | 3 | 4 | 5 | 6 |
|---|---|---|---|---|---|---|
| P₂O₅ | 70.2 | 67.4 | 64.6 | 61.8 | 59.0 | 60.5 |
| Na₂O | 29.8 | 28.6 | 27.4 | 26.2 | 25.0 | 39.5 |
| CaO | - | 4 | 8 | 12 | 16 | 0 |

As soluble glass, it is preferred to use a glass which has a solution or solubility rate of 0.1-1000 mg/cm²/hr at 25°C. The glass preferably has a saturation solubility at 25°C of at least 200 g/l, more preferably 800 g/l or greater, for phosphate glasses, and of at least 50 g/l for borate glasses.

The commonly available phosphate glasses are those from the binary system Na₂O:P₂O₅. The selection of glasses containing K₂O or mixed alkali metal oxides can be made on the same basis but glasses containing K₂O and/or mixtures of alkali metal oxides are less likely to be satisfactory as they are more prone to devitrification, and are also likely to be more costly.

A preferred glass is a phosphate glass from the binary system Na₂O:P₂O₅, with a molar ratio in the vicinity of 5Na₂O to 3P₂O₅. Although such glasses can vary slightly in composition, we have satisfactorily used a glass containing P₂O₅ 60.5 weight %, Na₂O 39.5 weight %. Such a glass has phosphate chains with an average value of n = 4.11, where n is the number of phosphate groups in the chain. We have carried out a variety of studies in order to assess the suitability of various water-soluble sodium polyphosphate glasses for use as binders. The following table shows compositions of some of the glasses tested:

| Glass Sample Number | Wt % P₂O₅ | Wt % Na₂O | Water |
|---|---|---|---|
| 1 | 69.0 | 30.5 | Balance |
| 2 | 67.0 | 32.5 | Balance |
| 3 | 65.0 | 34.5 | Balance |
| 4 | 63.0 | 36.5 | Balance |
| 5 | 60.5 | 39.0 | Balance |
| 6 | 58.0 | 41.5 | Balance |

We have noted that as the Na₂O content of the sodium polyphosphate glasses increases, the phosphate chain length generally becomes shorter and this in turn tends to increase the tensile strength of the product formed with the phosphate binder. We believe, without being bound by theory, that shorter phosphate chains may be better able to utilise hydrogen bonding and that the more chain end phosphate groups present may give stronger hydrogen bonding. We have also found with sodium polyphosphate glasses that as Na₂O content increases the dispersibility of a product employing such glasses as a binder tends to increase. We believe that this may indicate that the ability of partially hydrated glass to fully hydrate and dissolve into solution is affected by small changes in composition.

In addition, we have found that as the Na₂O content increases, the viscosity of the solution of the sodium polyphosphate glass in water also tends to increase. We believe that this tendency for an increase of viscosity may possibly indicate the tendency to have hydrogen bonding in aqueous solution. This in turn may possibly indicate that viscosity may indicate the suitability of a given sodium polyphosphate glass to be effective as a binder to give good solubility and tensile strength.

As specified hereinbefore, the glass must have a sufficiently high saturation solubility and solubility rate to enable it quickly and sufficiently to go into aqueous solution. We have found that all the glasses specified in the above Table have sufficient solubility rates and saturation solubility values.

Overall, it will be seen that there are a variety of factors which affect the choice and suitability of a binder. For any given application, the choice of a binder can be empirically determined by a trial and error technique.

Embodiments of the present invention will now be described.

In one embodiment of the invention the active material of a pesticide in dry powder form is mixed with an appropriate surfactant, a wetting agent and a dispersant. The mixture is then combined with 6% of a 50% by weight aqueous solution of a soluble glass of composition 70.2% by weight P₂O₅, 29.8% by weight Na₂O. This new mixture is pressed to form a briquette which is dried to remove excess water.

The drying operation is carried out by the use of either temperature or a vacuum depending on the heat stability of the active material.

The briquette so formed is relatively easy to transport and reduces the amount of contamination of its packaging by the active material.

The briquette will disperse almost immediately when placed in water, and the active material released forms a sprayable suspension which can be used directly on crops to destroy harmful insects and organisms.

In another embodiment of the invention the active material is of a pharmaceutical nature and in powder form. To this powder is added a 50% solution of phosphate glass in water at addition levels of 5, 10, 15, 20 and 25%. The powder/binder mixture is then wet massed. The mixture is then granulated by a conventional technique such as extrusion or wet granulation.

The granules can be pressed to form a tablet and depending on the glass composition the tablet will disperse almost immediately or over a period of time as desired. A fast dispersing tablet is made using a simple alkali phosphate glass as the binder and a slowly dispersing tablet is made using an alkali phosphate glass with added modifier ions such as calcium, magnesium or aluminium oxides. For example granules formed as aforedescribed were partially dried before pressing. Tabletting was carried out with conventional equipment at conventional settings. The tablets were then dried to remove excess water before the tablets were packed for storage.

Tablets formed from the above process dissolve almost instantaneously as do the granulae used to make them. Particularly good results were obtained at 15 and 20% addition levels to give strong tablets with fast dissolution rates.

A fast dispersing tablet can be used where a patient wishes to take a tablet without water. The tablet can be placed on the patient's tongue and will disperse rapidly in the normal saliva. Sweeteners or flavourings can be used as required.

A slow dispersing tablet can be used in applications where it is desirable for the active material to be dispersed over a relatively long period of time. For example the tablet may be in the form of a bolus which is then inserted into the rumen or stomach of an animal. The bolus will disperse over a period of time and thus the active material is dispersed over a period of time.

The principles of the invention may be used where it is desired to controllably release an active material where the active material is combined with a binder and the mixture can then be solidified. Examples of active materials suitable for use in the water-dispersible product of the invention include, in addition to pharmacologically-active substances and pesticides, herbicides and soil treatment agents, such as fertilizers, mineral supplements and soil pH adjustment agents. The water-dispersible products of the present invention may be prepared by the skilled person to have a predetermined dosage and/or application rate for the active material using common general knowledge in the art.

## Claims

1. A water dispersible product comprising at least one water-dispersible active material and a water-soluble binder, therefor wherein the water-soluble binder includes one or more of polyphosphate chains, borate ions or silicate ions.

2. A product according to claim 1, wherein the active material is selected from pharmacologically-active materials, pesticides and herbicides.

3. A product according to either claim 1 or claim 2, wherein the binder includes one or more of polyphosphate chains, borate ions or silicate ions which have been respectively derived from at least one water soluble phosphate and/or borate and/or silicate glass.

4. A product according to claim 3, wherein the water soluble phosphate glass comprises from 30 to 80 mol% P₂O₅, from 20 to 70 mol% R₂O, from 0 to 30 mol% MO and from 0 to 15 mol% L₂O₃ where R is Na, K or Li, M is Ca, Mg or Zn and L is Al, Fe or B.

5. A product according to claim 4, wherein the water soluble phosphate glass comprises from 58 to 72 wt% P₂O₅, from 42 to 28 wt% Na₂O and from 0 to 16 wt% CaO.

6. A product according to any one of claims 1 to 5 in the form of a tablet or a briquette.

7. A method of making the water-dispersible product of claim 1 comprising mixing at least one water-dispersible active material with the water-soluble binder in the form of an aqueous solution of a water soluble glass and then removing excess water.

8. A method of making the water-dispersible product of claim 1 comprising mixing at least one water-dispersible active material with particles of a water soluble glass, adding water to the mixture, allowing the water soluble glass to dissolve in the water and then removing excess water.

9. A method according to either claim 7 or claim 8, wherein the active material is selected from pharmacologically-active materials, pesticides and herbicides.

10. A method according to any one of claims 7 to 9, wherein the binder is derived from a water soluble phosphate glass comprising from 30 to 80 mol% P₂O₅, from 20 to 70 mol% R₂O, from 0 to 30 mol% MO and from 0 to 15 mol% L₂O₃ where R is Na, K or Li, M is Ca, Mg or Zn and L is Al, Fe or B.

11. A method according to claim 10, wherein the water soluble phosphate glass comprises from 58 to 72 wt% P₂O₅, from 42 to 28 wt% Na₂O and from 0 to 16 wt% CaO.

12. A method according to claim 7, wherein a pesticide in the form of dry powder is mixed with from 6 to 50% by weight of an aqueous solution of a soluble phosphate glass comprising 70.2% by weight P₂O₅ and 29.8% by weight Na₂O, the mixture is then pressed to form a briquette which is then dried to remove excess water.

13. A method according to claim 12, wherein at least one of a surfactant, a wetting agent or a dispersant is also mixed with the pesticide and water soluble glass solution.

14. A method according to claim 7, wherein a pharmacologically-active substance in dry powder form is mixed with a solution of a soluble phosphate glass and the mixture is then granulated and dried.

## Patentansprüche

1. In Wasser dispergierbares Produkt, umfassend mindestens ein in Wasser dispergierbares aktives Material und ein in Wasser lösliches Bindemittel dafür, wobei das wasserlösliche Bindemittel eine oder mehrere Polyphosphatketten, Borationen oder Silicationen enthält.

2. Produkt nach Anspruch 1, wobei das aktive Material ausgewählt ist aus pharmakologisch aktiven Materialien, Pestiziden und Herbiziden.

3. Produkt nach Anspruch 1 oder Anspruch 2, wobei das Bindemittel Polyphosphatketten, Borationen und/oder Silicationen enthält, die jeweils abgeleitet sind von mindestens einem in Wasser löslichen Phosphat- und/oder Borat- und/oder Silicatglas.

4. Produkt nach Anspruch 3, wobei das in Wasser lösliche Phosphatglas 30 bis 80 Mol-% P₂O₅, 20 bis 70 Mol-% R₂O und 0 bis 30 Mol-% MO und 0 bis 15 Mol-% L₂O₃ umfaßt, wobei R Na, K oder Li ist, M Ca, Mg oder Zn ist und L Al, Fe oder B.

5. Produkt nach Anspruch 4, wobei das in Wasser lösliche Phosphatlgas 58 bis 72 Gew.-% P₂O₅, 42 bis 28 Gew.-% Na₂O und 0 bis 16 Gew.-% CaO umfaßt.

6. Produkt nach einem der Ansprüche 1 bis 5 in Form einer Tablette oder eines Preßlings.

7. Verfahren zur Herstellung des in Wasser dispergierbaren Produktes nach Anspruch 1, umfassend das Vermischen von mindestens einem in Wasser dispergierbaren aktiven Material mit dem in Wasser löslichen Bindemittel in Form einer wäßrigen Lösung eines in Wasser löslichen Glases und anschließende Entfernung von Wasser.

8. Verfahren zur Herstellung des in Wasser dispergierbaren Produktes nach Anspruch 1, umfassend das Vermischen mindestens eines in Wasser dispergierbaren aktiven Materials mit Teilchen eines in Wasser löslichen Glases, Zugabe von Wasser zu dem Gemisch, Lösenlassen des in Wasser löslichen Glases in dem Wasser und anschließende Entfernung von überschüssigem Wasser.

9. Verfahren nach Anspruch 7 oder Anspruch 8, wobei das aktive Material ausgewählt ist aus pharmakologisch aktiven Materialien, Pestiziden und Herbiziden.

10. Verfahren nach einem der Ansprüche 7 bis 9, wobei das Bindemittel abgeleitet ist von einem in Wasser löslichen Phosphatglas, umfassend 30 bis 80 Mol-% P₂O₅, 20 bis 70 Mol-% R₂O, 0 bis 30 Mol-% MO und 0 bis 15 Mol-% L₂O₃, wobei R Na, K oder Li ist, M Ca, Mg oder Zn ist und L Al, Fe oder B.

11. Verfahren nach Anspruch 10, wobei das in Wasser lösliche Phosphatglas 58 bis 72 Gew.-% P₂O₅, 42 bis 28 Gew.-% Na₂O und 0 bis 16 Gew.-% Ca0 enthält.

12. Verfahren nach Anspruch 7, wobei ein Pestizid in Form von trockenem Pulver vermischt wird mit 6 bis 50 Gew.-% einer wäßrigen Lösung eines löslichen Phosphatglases, umfassend 70,2 Gew.-% P₂O₅ und 29,8 Gew.-% Na₂O, das Gemisch dann zu einem Preßkörper verpreßt wird, der dann getrocknet wird, um überschüssiges Wasser zu entfernen.

13. Verfahren nach Anspruch 12, wobei mindestens ein grenzflächenaktives Mittel, ein Netzmittel oder ein Dispergiermittel ebenfalls mit dem Pestizid und der Lösung des in Wasser löslichen Glases vermischt wird.

14. Verfahren nach Anspruch 7, wobei eine pharmakologisch aktive Substanz in trockener Pulverform vermischt wird mit einer Lösung eines löslichen Phosphatglases und das Gemisch dann granuliert und getrocknet wird.

## Revendications

1. Produit dispersible dans l'eau comprenant au moins un matériau actif dispersible dans l'eau et un liant soluble dans l'eau pour celui-ci, dans lequel le liant soluble dans l'eau contient une ou plusieurs des espèces suivantes : chaînes polyphosphate, ions borate ou ions silicate.

2. Produit selon la revendication 1, dans lequel le matériau actif est choisi parmi des matériaux pharmacologiquement actifs, des pesticides et des herbicides.

3. Produit selon l'une ou l'autre des revendications 1 ou 2, dans lequel le liant contient une ou plusieurs des espèces suivantes : chaînes polyphosphate, ions borate ou ions silicate, qui ont été respectivement obtenus à partir d'au moins un verre au phosphate et/ou au borate et/ou au silicate soluble dans l'eau.

4. Produit selon la revendication 3, dans lequel le verre au phosphate soluble dans l'eau contient de 30 à 80 moles % de P₂O₅, de 20 à 70 moles % de R₂O, de 0 à 30 moles % de MO et de 0 à 15 moles % de L₂O₃, dans lequel R est Na, K ou Li, M est Ca, Mg ou Zn, et L est Al, Fe ou B.

5. Produit selon la revendication 4, dans lequel le verre au phosphate soluble dans l'eau contient de 58 à 72% en poids de P₂O₅, de 42 à 28% en poids de Na₂O et de 0 à 16% en poids de Ca₂O.

6. Produit selon l'une quelconque des revendications 1 à 5, sous la forme d'une tablette ou d'une briquette.

7. Procédé pour préparer le produit dispersible dans l'eau selon la revendication 1, comprenant les étapes suivantes : mélanger au moins un matériau actif dispersible dans l'eau avec le liant soluble dans l'eau sous la forme d'une solution aqueuse d'un verre soluble dans l'eau et enlever ensuite l'excès d'eau.

8. Procédé de préparation du produit dispersible dans l'eau selon la revendication 1, comprenant les étapes suivantes : mélanger au moins un matériau actif dispersible dans l'eau avec des particules d'un verre soluble dans l'eau, ajouter de l'eau au mélange, laisser le verre soluble dans l'eau se dissoudre dans l'eau et enlever ensuite l'excès d'eau.

9. Procédé selon la revendication 7 ou la revendication 8, dans lequel le matériau actif est choisi parmi des matériaux pharmacologiquement actifs, des pesticides et des herbicides.

10. Procédé selon l'une quelconque des revendications 7 à 9, dans lequel le liant est dérivé d'un verre au phosphate soluble dans l'eau contenant de 30 à 80 moles % de P₂O₅, de 20 à 70 moles % de R₂O, de 0 à 30 moles % de MO et de 0 à 15 moles % de L₂O₃, dans lequel R est Na, K ou Li, M est Ca, Mg ou Zn, et L est Al, Fe ou B.

11. Procédé selon la revendication 10, dans lequel le verre au phosphate soluble dans l'eau contient de 58 à 72% en poids de P₂O₅, de 42 à 28% en poids de Na₂O et de 0 à 16% en poids de CaO.

12. Procédé selon la revendication 7, dans lequel un pesticide sous la forme d'une poudre sèche est mélangé avec 6 à 50% en poids d'une solution aqueuse d'un verre au phosphate soluble contenant 70,2% en poids de P₂O₅ et 29,8% en poids de Na₂O, le mélange est ensuite comprimé pour former une briquette qui est ensuite séchée pour enlever l'excès d'eau.

13. Procédé selon la revendication 12, dans lequel on mélange également au pesticide et à la solution de verre soluble dans l'eau au moins un agent parmi un agent tensioactif, un agent mouillant ou un dispersant.

14. Procédé selon la revendication 7, dans lequel une substance pharmacologiquement active sous forme de poudre sèche est mélangée avec une solution d'un verre au phosphate soluble et le mélange est ensuite granulé et séché.
